# EUROPÄISCHE PATENTSCHRIFT

(11) **EP 1 499 228 B1**
(45) Veröffentlichungstag und Bekanntmachung des Hinweises auf die Patenterteilung: **17.03.2010**
(21) Anmeldenummer: 04723193.1
(22) Anmeldetag: 25.03.2004
(51) Int. Cl.: A61B 1/00, G02B 6/028, G02B 23/26

(54) **ENDOSKOP**
ENDOSCOPE
ENDOSCOPE

(30) Priorität: 29.03.2003 DE 10314288
(43) Veröffentlichungstag der Anmeldung: 26.01.2005
(73) Patentinhaber: Schölly Fiberoptic GmbH, 79211 Denzlingen (DE)
(72) Erfinder: SCHLENKER, Stefan, 79111 Freiburg (DE)
(74) Vertreter: Maucher, Wolfgang
(86) Internationale Anmeldenummer: PCT/EP2004/003167
(87) Internationale Veröffentlichungsnummer: WO 2004/086954

(56) Entgegenhaltungen:
- EP-A- 1 229 359
- US-A- 2 975 785
- US-A- 4 813 400
- US-A- 5 443 057
- PATENT ABSTRACTS OF JAPAN vol. 011, no. 243 (P-603), 8. August 1987 (1987-08-08) & JP 62 052504 A (FUJIKURA LTD), 7. März 1987 (1987-03-07)

## Beschreibung

Die Erfindung bezieht sich auf ein Endoskop mit einem flexiblen Bildleiter, der in einem ihn umhüllenden Sondenschlauch verschieblich geführt und an seinen Endbereichen fixiert ist, wobei eine Fixierung zwischen Bildleiter und Sondenschlauch an einer ein Objektiv aufweisenden, distalen Sondenspitze und eine Fixierung am anderen, proximalen Sondenkopfende, des Bildleiters in einer ein Okular aufweisenden Halterung vorgesehen ist und zumindest der Bildleiter einen Ausgleichsabschnitt aufweist, in dem der Bildleiter als Schleife oder Teil einer Schleife verläuft.

Ein solches Endoskop bildet eine flexible Sonde. Die Bildaufnahme erfolgt durch ein Objektiv, welches sich an der Sondenspitze befindet. Das Objektiv ist direkt mit der Endfläche des Bildbündels verbunden und verklebt.
Die Bildübertragung von Objektiv zum Okular erfolgt mittels des flexiblen Bildleiters. Die Bildabnahme erfolgt am Sondenende an der Bildbündelendfläche durch ein Okular. Dabei befinden sich die Okularlinsen in einem definierten Luftabstand zur Bildbündelendfläche.
Das gesamte flexible Bildübertragungssystem, bestehend aus Objektiv und Bildbündel, befindet sich in dem Sondenschlauch. Der Sondenschlauch ist an der Sondenspitze über eine Verklebung mit dem Objektiv und dem Bildbündel verbunden und okularseitig ist der Schlauch mit der Halterung mittels Verklebung fixiert.
Wie bereits vorerwähnt, ist ein definierter Abstand zwischen der Bildbündelendfläche (Bildabnahmefläche) vorgesehen, um eine exakte Fokussierung und eine entsprechende Bildqualität zu erreichen.

Das flexible Bildübertragungssystem kann, bedingt durch unterschiedliche Ausdehnungskoeffizienten von Bildleiter und den ihn umhüllenden Sondenschlauch, in Abhängigkeit von den auftretenden Temperaturdifferenzen vergleichsweise hohen Schub- und Druckkräften ausgesetzt sein. Dies ist insbesondere dann der Fall, wenn das Endoskop im medizinischen Bereich eingesetzt wird und sterilisiert werden muss. Das Endoskop ist dabei Temperaturen im Bereich von etwa 130°C aufgesetzt.
Die auftretenden Relativverschiebungen zwischen Sondenschlauch und Bildleiter belasten dabei die Endbereiche, wo jeweils eine fixe Verbindung zwischen Sondenschlauch und Bildleiter vorgesehen ist. Der Sondenschlauch dehnt sich bei Erwärmung um ein materialabhängiges Maß stärker aus als der Bildleiter und wirkt mit einer entsprechenden Kraft auf die Verklebungen an den Enden des Bildleiter sowohl an der Sondenspitze als auch okularseitig.
Dies kann dazu führen, dass der fest vorgegeben Abstand für eine exakte Fokussierung im Bereich des Okulars am proximalen Ende verändert wird und dadurch Unschärfen innerhalb der Bildübertragung auftreten können.
Auch kann diese Ausdehnungskraft den Bildleiter aus seiner fixierten Lage an der Sondenspitze oder auch im Okularbereich herausziehen oder aber es kommt aufgrund der auftretenden Kräfte zum Bruch des Bildbündels. Solche Beschädigungen machen das Endoskop unbrauchbar.

Die US 4,813,400 zeigt ein Endoskop mit einer Sonde und einer ein okular aufweisenden Halterung und einem dazwischen fest angeordneten Bildleiter. Damit beim Benutzen der Sonde keine Spannungen auf den fest verbundenen Bildleiter übertragen werden, weist der Bildleiter einen mäanderartigen Abschnitt oder eine Schleife auf. Dadurch werden Biegemomente sowie Druck- und Schubkräfte nicht an die Okular aufweisende Halterung übertragen und diese vor Beschädigung geschützt.

Aufgabe der vorliegende Erfindung ist es, ein Endoskop der eingangs erwähnten Art zu schaffen, das auch unter dem Einfluss erheblicher Temperaturschwankungen betriebssicher ist und bei dem Beschädigungen bei einer hohen Temperaturbeaufschlagung, wie sie zur Sterilisation erforderlich sind, sicher vermieden werden.

Zur Lösung dieser Aufgabe wird vorgeschlagen, dass der den Bildleiter umhüllende Sondenschlauch vor der proximalen Fixierung des Bildleiters durch die das okular, Halterung endet, dass die Schleife oder der Teil einer Schleife in einem Gehäuse angeordnet ist und sich innerhalb des Gehäuses bereichsweise an der Gehäuseinnenwand, insbesondere an etwa gegenüberliegenden Seiten abstützt und dadurch in eine von der Kreisform abweichende Form, insbesondere oval, geformt ist und dass der Sondenschlauch distalseitig vor diesem Abschnitt relativ zu der proximalen Okularhalterung des Bildleiters fixiert ist.

Durch die von der gestreckten Lage abweichende geometrische Form des flexiblen Bildleiters ist eine Dehnungsschleife gebildet, die es ermöglicht, die auftretenden, unterschiedlichen Längenausdehnungen von Bildleiters und Sondenschlauch auszugleichen. In der gestreckten Lage ist der flexiblen Bildleiter spannungsfrei und es sind praktisch keine ihn aus dieser Lage verändernden Rückstellkräfte aktiv. Die gestreckte Lage bildet somit nicht zwangsläufig eine absolute Gerade.
Der für die Funktionsweise der flexiblen Sonde erforderliche Aufbau mit Fixierungen an den Enden wird durch die erfindungsgemäße Maßnahme praktisch vollständig oder zumindest erheblich geringer belastet, so dass die bislang negativen Auswirkungen der unterschiedlichen Ausdehnungen, zum Beispiel das Herausziehen des Bildleiters aus seinen verklebungen an den Enden und die damit verbundene Nutzungseinschränkung beziehungsweise der Funktionsausfall dadurch vermieden werden. Durch die Fixierung des Sondenschlauchs distalseitig vor dem Abschnitt mit dem von der gestreckten Form abweichenden Verlauf des Bildleiters relativ zu der proximalen Halterung des Bildleiters ist ein dichter Abschluss des flexibel beweglichen Sondenteils gebildet.
Der Sondenschlauch kann einerseits an der Befestigungsstelle distalseitig vor dem Ausgleichsabschnitt engen, andererseits könnte er sich aber auch noch über den Abschnitt mit dem von der gestreckten Form abweichenden Verlauf des Bildleiters oder über einen Teilbereich dieses Abschnittes erstrecken.
Der Platzbedarf für die "Dehnungsschleife" des Halbleiters ohne Ummantelung durch den Sondenschlauch ist jedoch wesentlich geringer. Bei mitlaufendem Sondenschlauch ist der Bildleiter besser geschützt. Die praktizierte Ausführungsform wird demnach durch die jeweiligen, konstruktiven Vorgaben bestimmt.
Um die insbesondere bei großen Temperaturschwankungen auftretenden Relativverschiebungen von Sondenschlauch und Bildleiter möglichst klein zu halten, ist grundsätzlich anzustreben Materialien zu verwenden, deren Ausdehnungskoeffizienten einen möglichst geringen Unterschied, im Idealfall etwa gleiche Ausdehnungskoeffizienten aufweisen, um diesbezügliche Probleme zu vermeiden. In der Praxis ist durch die sehr unterschiedlichen Anforderungen einerseits an den Bildleiter und andererseits an den Sondenschlauch und unter Berücksichtigung der unter anderem kostengünstig zur Verfügung stehenden Materialpaarungen, eine Anpassung der Ausdehnungskoeffizienten nur unvollständig möglich, so dass noch erheblich unterschiedliche Längenänderungen auftretenden können. Durch die erfindungsgemäße Maßnahmen werden jedoch die sonst gravierend nachteiligen, eingangs beschrieben Auswirkungen eliminiert.

Dadurch dass sich an das proximale Ende des Abschnitts mit dem von der gestreckten Form abweichenden Verlauf zumindest des Bildleiters praktisch unmittelbar die Halterung für den Bildleiter anschließt, wird eine kompakte Bauform begünstigt und der sich distalseitig anschließende, flexible Sondenteil steht praktisch in voller Länge zur Verfügung.
Dabei ist es vorteilhaft, wenn die distalseitig vor dem Abschnitt mit dem von der gestreckten Form abweichenden Verlauf vorgesehene Fixierung des Sondenschlauchs sowie die proximale Halterung des Bildleiters bei dem Okulars, Teil des Gehäuses sind.
Es ist dadurch am okularseitigen Ende des Endoskops eine kompakte Einheit als Sondenkopf gebildet. Besonders vorteilhaft ist hierbei auch, dass insgesamt ein geschlossenes System gebildet ist, so dass von außen nichts eindringen kann. Dies ist insbesondere im medizinischen Bereich eine wichtige Voraussetzung und in Verbindung mit dem Sterilisieren durch Autoklavieren von wesentlicher Bedeutung.

Die windungsartige geometrische Form bildet platzsparend eine Dehnungsschleife, die auch große temperaturbedingte und materialbedingte Relativverschiebungen zwischen Bildleiter und Sondenschlauch ausgleichen kann.
Je nach Sondenlänge kann die Schleife oder Windung mit einer oder mehreren Windungen ausgeführt sein, wobei bei kurzen Sonden auch eine Teilwindung mit einem etwa U-förmigen Verlauf genügt.
Dabei verläuft die schleife oder Teil-Schleife etwa in einer zur Längserstreckung des Bildleiters parallelen Ebene, so dass bei Dehnungs-Ausgleichsbewegungen insbesondere eine Torsion des Bildleiters vermieden wird.
Nach einer Ausgestaltung der Erfindung kann zumindest eine Biegung des Bildleiters in dem Abschnitt mit einem von der gestreckten Form abweichenden Verlauf in Biegeposition thermisch fixiert sein.

Der Bildleiter ist gegenüber Auslenkungen aus einer gestreckten Lage federelastisch, so dass bei einer Schleifenbildung Rückstellkräfte auftreten, die allerdings nur so groß sind, dass sie von den fixierten Sondenenden ohne Gefahr einer Beschädigung aufgenommen werden können. Um jedoch auch diese Krafteinwirkung zu reduzieren oder zu vermeiden kann eine thermische Fixierung der Ausgleichs-Schleife vorgenommen werden. Je nach geometrischem Verlauf kann sich diese thermische Fixierung über die gesamte Schleife oder nur über einen Teilbereich erstrecken.
Die thermische Fixierung wird vorgenommen, indem die zu fixierende Stelle soweit erwärmt wird, bis die elastische Spannung in diesem Bereich reduziert oder zumindest teilweise beseitigt ist.

Zweckmäßigerweise weist das Gehäuse für den Bildleiter etwa an gegenüberliegenden Seiten einen Zugang mit Fixierung des Sondenschlauchs und einen Abgang mit Halterung des Bildleiters auf, wobei die Anlagestellen an der Innenwand des Gehäuses etwa mittig zwischen Zu - und Abgang liegen.
Durch die Verformung der Schleife aus einer Kreisform in eine ovale Form durch eine entsprechende Eingrenzung und Beaufschlagung der Schleife innerhalb des Gehäuses ist es zum einen ermöglicht, dass die auftretender Kräfte in Form von Reibkräften an den kräfteunkritischen Berührungsflächen zur Gehäusewand abgeleitet werden und zum anderen kann durch die ovale Schleife, insbesondere bei langen Sonden, Temperaturdifferenzen entstehende, unterschiedliche Längenausdehnung der Konstruktionskomponenten, insbesondere des Sondenschlauchs und des Bildleiters, kompensiert werden. Durch die Form des Ovals ist auch beim Zusammenziehen der Schleife gewährleistet, dass das Bildbündel an der Wand des insbesondere durch eine PTFE-Hülse gebildeten Gehäuses anliegt und somit eine Kraftübertragung auf das Gehäuse erfolgen kann.

Anstatt in einer ovalen Schlaufe kann der Bildleiter zum Dehnungsausgleich in einem U-förmigen Bogen verlaufen. In diesem Fall ist es besonders zweckmäßig, den Bildleiter bei den Biegestellen thermisch zu fixieren, weil dann der Bildleiter in Richtung der Bildbündelachse unbelastet und kräftefrei ist. Ein U-förmigen Bogen des Bildleiters kann insbesondere bei kürzeren Sonden, wo nur geringe Längenänderungen auszugleichen sind, vorgesehen sein.
Mit den vorbeschriebenen Geometrien sind die kräftekritischen Bereiche der Verklebungen des flexiblen Bildübertragungssystems praktisch nahezu kräftefrei.
Außerdem ist es durch die geometrische Form des Ovals bzw. der U-Form möglich, den Bildleiter praktisch torsionsfrei zu verbauen, womit die Bildlage, im Gegensatz zu einem tordierten Bildbündel, unbeeinflusst bleibt.

Eine vorteilhafte Ausgestaltung der Erfindung sieht vor, dass der Bildleiter aus einem Quarzbildbündel von Stufenindexfasern besteht, die jeweils ein Kernglas und ein Mantelglas aufweisen und dass im Verlauf des Bildleiters, insbesondere in dem Abschnitt mit einem von der gestreckten Form abweichenden Verlauf, wenigstens eine bogenförmige Knickstelle vorgesehen ist, deren Biegeradius derart bemessen ist, dass innerhalb des Mantels geführte Lichtstrahlen im Bereich der Knickstelle nach außen aus der Einzelfaser austreten.
Zweckmäßigerweise verhält sich dabei im Bereich einer bogenförmigen Knickstelle der Biegeradius des Quarzbildbündels zu dem Durchmesser der Einzelfaser etwa wie 100 zu 1 bis 5000 zu 1.
Durch diese Abwinkelung des Quarzbildbündels wird eine deutliche Kontraststeigerung erzielt. Dies beruht darauf, dass Strahlen, welche sich genau unter dem Grenzwinkel der Totalreflexion oder wenig darüber in der Einzelfaser ausbreiten, die Abwinkelung nicht passieren können, da für diese Strahlen die Totalreflexionsbedingung nicht gegeben sind. Diese Strahlen werden gebrochen und verlassen das Quarzbildbündel senkrecht zur Ausbreitungsrichtung.
Damit wird verhindert oder zumindest reduziert, dass Licht und damit Informationen von einer Faser zu den nächsten Nachbarfasern übertritt. Das sogenannte Übersprechen, also die unerwünschte gegenseitige Beeinflussung benachbarter Informationskanäle (Einzelfasern) wird dadurch reduziert und damit der Kontrast verbessert.
Die bogenförmige Knickstelle kann mechanisch durch eine Halterung und/oder thermisch fixiert sein. Bei einer thermischen Fixierung wird der bogenförmige Verlauf nach einer für eine plastische Verformung ausreichenden Erwärmung des Bildleiters fixiert, so dass anschließend in diesem Bereich federelastische Rückstellkräfte eliminiert sind.
Weiterhin kann auch eine Kombination aus beiden Maßnahmen zur Lagefixierung vorgesehen sein.
Durch die Lagefixierung bleibt die bogenförmige Knickstelle mit ihrer zur Kontrastverbesserung notwendigen, geometrischen Form der Knickstelle auch bei mechanischer Belastung erhalten.
Die zur Kontrastverbesserung vorgesehene Knickstelle kann in vorteilhafter Weise Teil des Bildleiter-Abschnittes sein, der einen von der gestreckten Form abweichenden Verlauf, also eine windungsartige Schlaufenform oder einen U-förmigen Verlauf zum Dehnungsausgleich aufweist. Damit hat dieser Dehnungsausgleichs-Abschnitt eine Mehrfachfunktion.
Gegebenenfalls kann im Verlauf des Bildleiters, vorzugsweise zusätzlich zu wenigstens einer bogenförmigen Knickstelle, wenigstens eine Torsionsstelle vorgesehen sein, bei der die Einzelfasern schraubenlinienförmig verlaufen. Diese Torsionsstelle(n) kann vorzugsweise unmittelbar benachbart zu einer Knickstelle angeordnet sein.
In erster Linie kann damit eine Lagekorrektur der Einzelfasern vorgenommen werden, um eine lagegerechte Bildzuordnung der Bildleiter-Enden zu erreichen. Wenn sich die Torsionsstelle(n) bei einer Knickstelle, insbesondere unmittelbar benachbart dazu befindet, kann diese bei der dort vorgesehenen Lagefixierung vorteilhafterweise gleich mit lagefixiert sein. Eine solche Torsionsstelle kann zusätzlich auch zur Verbesserung des Kontrastes dienen und damit gegebenenfalls die Kontraststeigerung durch die erfindungsgemäße Maßnahme unterstützen.

Nachstehend ist die Erfindung mit ihren wesentlichen Einzelheiten anhand der Zeichnungen noch näher erläutert.

Es zeigt:
- Fig. 1: eine etwas schematisierte Darstellung eines Endoskops mit einem Längen-Ausgleichsabschnitt,
- Fig. 2: eine vergrößerte Detaildarstellung einer Sondenspitze des Endoskops im Querschnitt,
- Fig. 3: eine etwa Fig. 1 entsprechende Darstellung, hier jedoch mit einer abgewandelten Ausführungsform eines Längen-Ausgleichsabschnittes und
- Fig. 4: eine schematische Darstellung eines Bildleiters mit an den Enden erkennbaren Bildflächen.

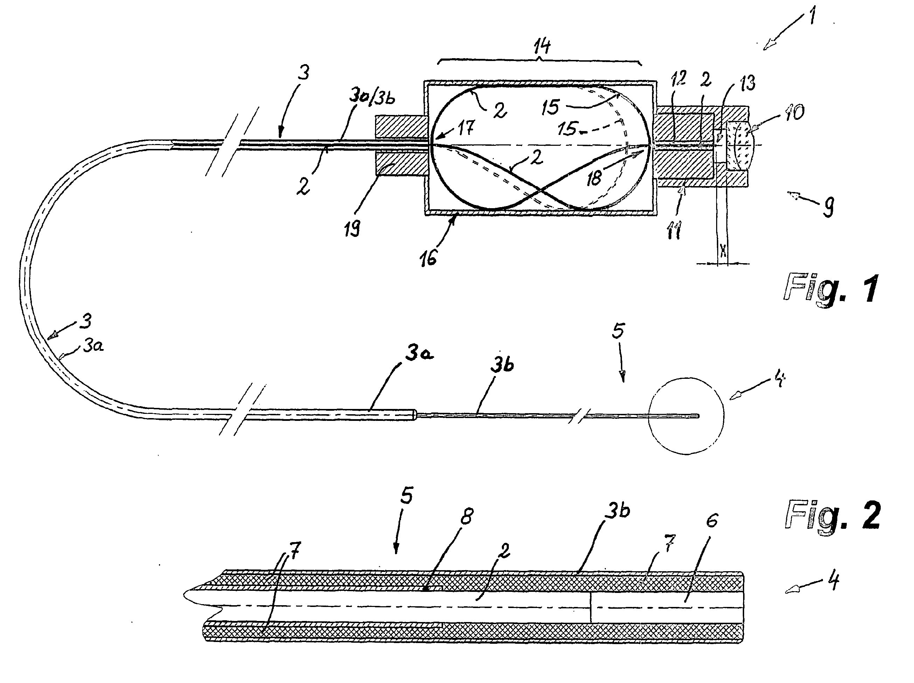

Ein in Fig. 1 gezeigtes Endoskop 1 weist einen flexiblen Bildleiter 2 auf, der in einem ihn umhüllenden Sondenschlauch 3 verschieblich geführt ist. Im Ausführungsbeispiel ist der Sondenschlauch 6 zweischichtig aus einem Außenschlauch 3a und einem dünneren Innenschlauch 3b gebildet, die miteinander verbunden sind. Beim distalen Ende 4 tritt der Innenschlauch 3b aus dem Außenschlauch 3a aus und ist dort beispielsweise 10 bis 20 cm weitergeführt. Wegen des geringeren Außenquerschnittes lässt sich die Sondenspitze 5 auch an schwer zugänglichen Stellen positionieren. Im übrigen Sondenbereich sorgt die etwas dickere Ummantelung, bestehend aus Innenschlauch 3b und Außenschlauch 3a für einen guten Schutz des darin geführten Bildleiters 2 und für eine bessere Handhabbarkeit.

Im Bereich des distalen Endes 4 endet der Bildleiter 2 und es schließt sich unmittelbar ein Objektiv 6 an (Fig.2). Der Bildleiter 2 und das Objektiv 6 sind mittels Kleber 7 mit dem Innenschlauch 3b des Sondenschlauches 3 verbunden. In Fig. 2 ist auch noch gut erkennbar, dass der Bildleiter 2 außenseitig eine Schutzlack-Beschichtung 8 trägt, die vor der Übergangsstelle zu dem Objektiv 6 endet.

Am proximalen Ende 9 ist der Bildleiter 2 in einer ein Okular 10 aufweisenden Halterung 11 fixiert, was durch eine dichte Verklebung 12 erreicht wird. Das dort befindliche Ende des Bildleiters befindet sich mit seiner Bildbündelendfläche 13 in einem definierten Luftabstand X zu den Linsen des Okulars 10.

Wie gut in Fig. 1 erkennbar, endet der den Bildleiter 2 umhüllende Sondenschlauch 3 beabstandet zu der proximalen Fixierung des Bildleiters 2 in der Halterung 11, wobei das Schlauchende relativ zu der proximalen Okularhalterung 11 des Bildleiters fixiert ist. Zwischen dieser Fixierung des Sondenschlauches 3 und der Halterung 11 des Bildleiters 2 befindet sich ein Ausgleichsabschnitt 14, in dem der Bildleiter einen von der gestreckten Form abweichenden Verlauf aufweist.

Im Ausführungsbeispiel gemäß Fig. 1 ist der Bildleiter in einer einwindigen Schlaufe 15 geführt. Diese befindet sich innerhalb eines Gehäuses 16, das an einem Ende einen Zugang 17 und gegenüberliegend am anderen Ende einen Abgang 18 für den Bildleiter 2 aufweist. An den Abgang 18 schließt sich die Halterung 11 zum Fixieren des Bildleiterendes sowie zur Halterung des Okulars 10 an und an den Zugang 17 auf der gegenüberliegenden Seite ist der Sondenschlauch 3 in einer Schlauchaufnahme 19 fixiert. Der Schlauch ist dabei in der als Buchse ausgebildeten Schlauchaufnahme 19 dicht verklebt.

Das Gehäuse 16 und die Größe der Schlaufe 15 sind so bemessen, dass die Schlaufe aus einer Kreisform zu einer etwa ovalen Form verformt wird. Es sind somit an der Gehäuseinnenwand Anlagestellen vorhanden, an denen die auftretenden Kräfte durch die Elastizität des Bildleiters 2 in Form von Reibkräften an den Berührungsflächen zur Gehäuseinnenseite abgeleitet werden. Dadurch werden durch die Auffedertendenz der Schlaufe vorhandene Rückstellkräfte kompensiert und wirken sich nicht oder zumindest reduziert auf die Fixierungen des Bildleiters 2 an seinen Ende aus.

Wird das Endoskop 1 einer erhöhten Temperatur, zum Beispiel beim Autoklavieren, ausgesetzt, dehnt sich der Sondenschlauch 3 wesentlich weiter aus als der Bildleiter 2. Da der Bildleiter 2 verschiebbar zwischen den beiden Fixierstellen innerhalb des Sondenschlauches geführt ist, kann ein der Ausdehnung des Sondenschlauches 3 entsprechender Abschnitt des Bildleiters 2 aus der Schlaufe 15 nachgezogen werden, wobei sich die Schlaufe 15, wie strichliniert angedeutet, etwas verkleinern kann. Auf die Fixierenden des Bildleiters 2 einwirkende Zugkräfte werden dadurch sicher vermieden.

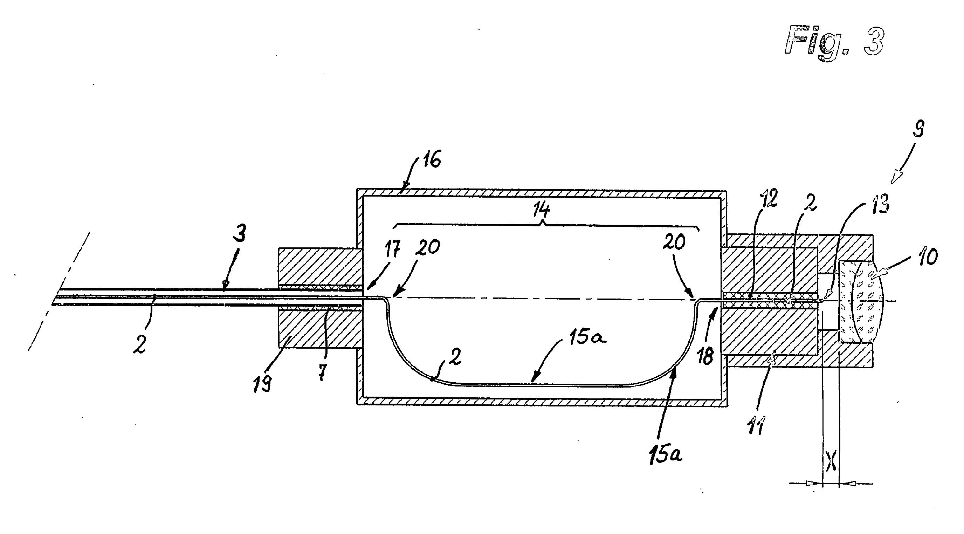

Fig. 3 zeigt eine andere Ausführungsform eines Ausgleichsabschnittes 14, wobei hier anstatt einer ein- oder mehrwindigen Schlaufe nur eine Teilschlaufe 15a mit einem im wesentlichen U-förmigen Verlauf vorgesehen ist. Bei Längenänderungen des Sondenschlauches relativ zu dem Bildleiter 20 kann sich die U-Weite entsprechend aufweiten oder verengen. In jedem Fall werden auch hier aus Dehnungsunterschieden des Bildleiters und des Sondenschlauches resultierende Längenänderungen über die U-Teilschlaufe 15a abgebaut, ohne dass Zug- und Druckspannungen auf die Fixierungen des Bildleiters an dessen Enden einwirken.

Ebenso wie bei der Ausführungsform nach Fig. 1 besteht die Möglichkeit, die Schlaufe 15 oder die Teilschlaufe 15a thermisch so zu behandeln, dass eine Fixierung einer mittleren Lage erfolgt und dabei insbesondere die federelastische Spannung entfällt. Gerade bei der Ausführungsform nach Fig. 3 würden sich durch die Federelastizität des Bildleiters Schubkräfte aufbauen, die zwar vergleichsweise gering sind und die Fixierungen an den Enden des Bildleiters 2 nicht gefährden, jedoch kann durch eine thermische Fixierung auch diese Kraftbeaufschlagung vermieden werden.

Innerhalb der Schlaufe 15 oder der Teilschlaufe 15a oder an einem Übergangsbereich dazu kann eine spezielle Formung des Bildleiters vorgesehen sein, um eine Kontrasterhöhung zu erzielen. Dies wird erreicht, indem bei einem Bildleiter aus einem Quarzbildbündel von Stufenindexfasern, die jeweils ein Kernglas und ein Mantelglas aufweisen, wenigstens eine bogenförmige Knickstelle 20 vorgesehen ist, deren Biegeradius derart bemessen ist, dass innerhalb des Mantels geführte Lichtstrahlen im Bereich der Knickstelle 20 nach außen aus den Einzelfasern austreten.
Durch das Ableiten des im Mantel geführten Lichtes wird verhindert, dass Licht und damit Informationen von einer Faser zu den nächsten Nachbarfasern übertreten, wodurch der Kontrast verbessert wird. Im Ausführungsbeispiel gemäß Fig. 3 können die zur Bildung der U-förmigen Teilschlaufe sowieso erforderlichen Knickstellen so modifiziert sein, dass diese Auskoppelbedingungen gegeben sind. Durch eine thermische Fixierung bleibt der passende Knickwinkel erhalten und damit die optischen Voraussetzungen zum Austreten der Lichtstrahlen aus dem Mantel.

Es besteht weiterhin die Möglichkeit, im Verlauf des Bildleiters, vorzugsweise zusätzlich zu wenigstens einer bogenförmigen Knickstelle 20, wenigstens eine Torsionsstelle vorzusehen, bei der die Einzelfasern schraubenlinienförmig verlaufen.

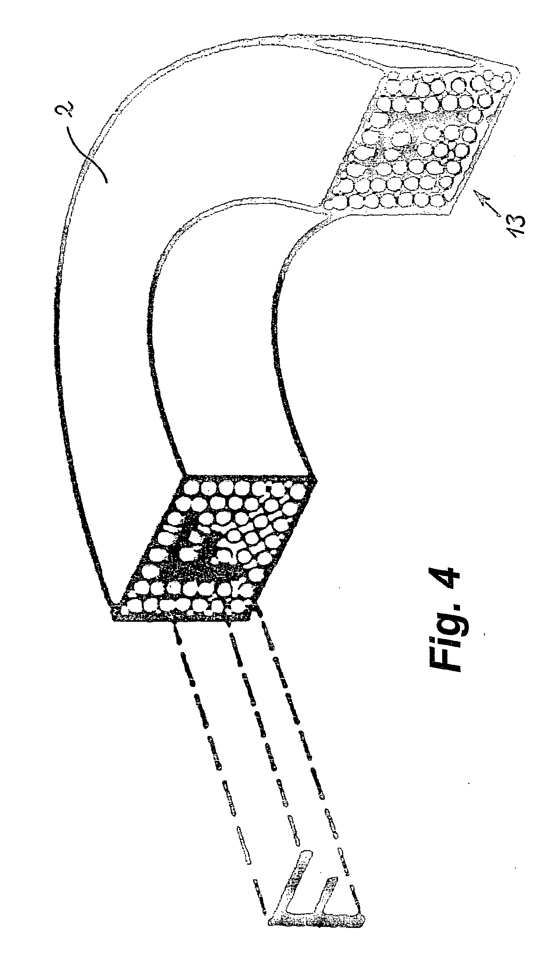

Wie in Fig. 4 erkennbar, ist es erforderlich, über den Verlauf des Bildleiters 2 die Einzelfasern so zu führen, dass ein Bild lagerichtig übertragen wird. Durch eine oder mehrere Torsionsstellen kann eine entsprechende Lagekorrektur vorgenommen werden. Darüber hinaus kann auch mit einer solchen Torsionsstelle in gewissem Maße eine Erhöhung des Kontrastes erreicht werden. Sind zwei solcher Torsionsstellen vorgesehen, beispielsweise jeweils eine im Bereich der Knickstellen 20, so kann die exakte Bildzuordnung an den Enden des Bildleiters 2 durch unterschiedliche Torsionsrichtungen erreicht werden.

## Patentansprüche

1. Endoskop mit einem flexiblen Bildleiters, der in einem ihn umhüllenden Sondenschlauch verschieblich geführt und an seinen Endbereichen fixiert ist, wobei eine Fixierung zwischen Bildleiter und Sondenschlauch an einer ein Objektiv aufweisenden, distalen Sondenspitze und eine Fixierung am anderen, proximalen Sondenkopfende des Bildleiters in einer ein Okular aufweisenden Halterung vorgesehen ist und zumindest der Bildleiter (2) einen Ausgleichsabschnitt (14) aufweist, in dem der Bildleiter (2) als Schleife (15) oder Teil einer Schleife (15a) verläuft, wobei der den Bildleiters (2) umhüllende Sondenschlauch (3) vor der proximalen Fixierung das Bildleiters (2) durch die das Okular (10) aufweisende Halterung (11) endet, der Sondenschlauch (3) distalseitig vor diesem Abschnitt relativ zu der proximalen Okularhalterung (11) des Bildleiters (2) fixiert ist, die Schleife (15) oder der Teil einer Schleife (15a) in einem Gehäuse (16) angeordnet ist, **dadurch gekennzeichnet, dass** sich die Schleife (15) oder der Teil der Schleife (15) innerhalb des Gehäuses (16) bereichsweise an der Gehäuseinnenwand, insbesondere an etwa gegenüberliegenden Seiten abstützt und dadurch in eine von der Kreisform abweichende Form, insbesondere oval, geformt ist.

2. Endoskop nach Anspruch 1 , **dadurch gekennzeichnet, dass** sich an das proximale Ende (9) des Ausgleichabschnitts (14) praktisch unmittelbar die Halterung (11) für den Bildleiter (2) anschließt.

3. Endoskop nach Anspruch 1 oder 2, **dadurch gekennzeichnet, dass** die distalseitig vor dem Ausgleichsabschnitt vorgesehene Fixierung (19) des Sondenschlauchs (3) sowie proximale Halterung (11) des Bildleiters (2) bei dem Okular(10) Teil des Gehäuses (16) sind.

4. Endoskop nach einem der Ansprüche 1 bis 3, **dadurch gekennzeichnet, dass** die Schleife (15) oder Teil-Schleife (15a) etwa in einer zur Längserstreckung des Bildleiters (2) parallelen Ebene verläuft.

5. Endoskop nach einem der Ansprüche 1 bis 4, **dadurch gekennzeichnet, dass** die schleife (15) oder Teil-Schleife (15a) des Bildleiter (2) bereichsweise mit dem Sondenschlauch (3) umhüllt ist.

6. Endoskop nach einem der Ansprüche 1 bis 5, **dadurch gekennzeichnet, dass** der Sondenschlauch (3) an der Befestigungsstelle distalseitig vor der Schleife (15) oder der Teil-Schleife (15a) des Bildleiters (2) endet.

7. Endoskop nach einem der Ansprüche 1 bis 6, **dadurch gekennzeichnet, dass** das Gehäuse (16) für den Bildleiter (2) etwa an gegenüberliegenden Seiten einen Zugang (17) mit Fixierung des Sondenschlauchs (3) und einen Abgang (18) mit Halterung des Bildleiters (2) aufweist und dass die Anlagestellen an der Innenwand des Gehäuses (16) etwa mittig zwischen Zu- und Abgang liegen.

8. Endoskop nach einem der Ansprüche 1 bis 7, **dadurch gekennzeichnet, dass** das im Sondenkopfbereich vorgesehene Gehäuse (16) durch eine Hülse aus zumindest innenseitig gleitfähigem Material, vorzugsweise aus PTFE gebildet ist.

9. Endoskop nach einem der Ansprüche 1 bis 8, **dadurch gekennzeichnet, dass** zumindest eine Biegung des Bildleiter (2) in dem Abschnitt mit einem von der gestreckten Form abweichenden Verlauf, in Biegeposition thermisch fixiert ist.

10. Endoskop nach einem der Ansprüche 1 bis 9, **dadurch gekennzeichnet, dass** der Bildleiter (2) aus einem Quarzbildbündel von Stufenindexfasern besteht, die jeweils ein Kernglas und ein Mantelglas aufweisen und dass im Verlauf des Bildleiters (2), insbesondere in dem Abschnitt mit einem von der gestreckten Form abweichenden Verlauf, wenigstens eine bogenförmige Knickstelle (20) vorgesehen ist, deren Biegeradius derart bemessen ist, dass innerhalb des Mantels geführte Lichtstrahlen im Bereich der Knickstelle (20) nach außen aus den Einzelfasern austreten.

11. Endoskop nach Anspruch 10, **dadurch gekennzeichnet, dass** sich im Bereich einer bogenförmigen Knickstelle (20) der Biegeradius des Quarzbildbündels zu dem Durchmesser der Einzelfaser etwa wie 100 zu 1 bis 5000 zu 1 verhält.

12. Endoskop nach Anspruch 10 oder 11, **dadurch gekennzeichnet, dass** die bogenförmige Knickstelle (20) mechanisch durch eine Halterung und/oder thermisch fixiert ist.

13. Endoskop nach einem der Ansprüche 1 bis 12, **dadurch gekennzeichnet, dass** im Verlauf des Bildleiters (2) vorzugsweise zusätzlich zu wenigstens einer bogenförmigen Knickstelle (20) wenigstens eine Torsionsstelle vorgesehen ist, bei der die Einzelfasern schraubenlinienförmig verlaufen.

14. Endoskop nach Anspruch 13, **dadurch gekennzeichnet, dass** sich die Torsionsstelle(n) bei einer Knickstelle (20), insbesondere unmittelbar benachbart dazu befindet.

## Claims

1. Endoscope having a flexible image conductor which is movably guided in a probe tube that encases it and is secured in its end regions, wherein a fixing is provided between the image conductor and the probe tube at a distal probe tip that comprises an objective and a fixing is provided at the other, proximal probe head end of the image conductor in a holder that comprises an eyepiece, and at least the image conductor (2) has a compensating section (14) in which the image conductor (2) extends as a loop (15) or part of a loop (15a), wherein the probe tube (3) that encases the image conductor (2) terminates before the proximal fixing of the image conductor (2) through the holder (11) that comprises the eyepiece (10), the probe tube (3) is fixed at the distal end before this section relative to the proximal eyepiece holder (11) of the image conductor (2), the loop (15) or the part of a loop (15a) is arranged in a housing (16), **characterised in that** the loop (15) or the part of the loop (15) is supported at places within the housing (16) on the inner wall of the housing, in particular on roughly opposite sides, and is thereby shaped into a form that is other than circular, in particular oval.

2. Endoscope according to claim 1, **characterised in that** the holder (11) for the image conductor (2) is virtually immediately adjacent to the proximal end (9) of the compensating section (14).

3. Endoscope according to claim 1 or 2, **characterised in that** the fixing (19) of the probe tube (3) provided at the distal end before the compensating section and the proximal holder (11) of the image conductor (2) at the eyepiece (10) are part of the housing (16).

4. Endoscope according to one of claims 1 to 3, **characterised in that** the loop (15) or partial loop (15a) extends substantially in a plane that is parallel to the longitudinal extent of the image conductor (2).

5. Endoscope according to one of claims 1 to 4, **characterised in that** the loop (15) or partial loop (15a) of the image conductor (2) is partially encased in the probe tube (3).

6. Endoscope according to one of claims 1 to 5, **characterised in that** the probe tube (3) terminates at the fixing point at the distal end before the loop (15) or the partial loop (15a) of the image conductor (2).

7. Endoscope according to one of claims 1 to 6, **characterised in that** the housing (16) for the image conductor (2) comprises, on opposite sides, an entrance (17) with securing of the probe tube (3) and an exit (18) with securing of the image conductor (2), and **in that** the abutment points on the inner wall of the housing (16) are located substantially centrally between the entrance and exit.

8. Endoscope according to one of claims 1 to 7, **characterised in that** the housing (16) provided in the probe head region is formed by a sleeve of material that is slippery at least on the inside, preferably PTFE.

9. Endoscope according to one of claims 1 to 8, **characterised in that** at least one bend in the image conductor (2) is thermally fixed in the bent position in the portion that has a configuration deviating from the extended shape.

10. Endoscope according to one of claims 1 to 9, **characterised in that** the image conductor (2) consists of a quartz image bundle of step index fibres each of which has a core glass and a casing glass and **in that** along the course of the image conductor (2), particularly in the portion that has a configuration deviating from the extended shape, at least one arcuate kink (20) is provided, the bending radius of which is of such dimensions that light beams guided within the casing emerge outwards from the individual fibres in the region of the kink (20).

11. Endoscope according to claim 10, **characterised in that** in the region of an arcuate kink (20) the bending radius of the quartz image bundle is to the diameter of the individual fibre in the ratio 100 to 1 to 5000 to 1.

12. Endoscope according to claim 10 or 11, **characterised in that** the arcuate kink (20) is fixed mechanically by a clamp and/or thermally.

13. Endoscope according to one of claims 1 to 12, **characterised in that** along the course of the image conductor (2) preferably in addition to at least one arcuate kink (20) at least one torsion point is provided at which the individual fibres run helically.

14. Endoscope according to claim 13, **characterised in that** the torsion point(s) is or are located at a kink (20), in particular immediately adjacent thereto.

## Revendications

1. Endoscope comprenant un transmetteur d'image flexible qui est guidé de manière coulissante dans un flexible de sonde par lequel il est enveloppé et qui est fixé à ses zones d'extrémité, une fixation entre transmetteur d'image et flexible de sonde à une pointe de sonde distale présentant un objectif et une fixation à l'autre extrémité de tête de sonde, proximale, du transmetteur d'image dans un support présentant un oculaire étant prévues, et au moins le transmetteur d'image (2) présentant une section de compensation (14) dans laquelle le transmetteur d'image (2) s'étend sous forme de boucle (15) ou de partie d'une boucle (15a), le flexible de sonde (3) qui enveloppe le transmetteur d'image (2) se terminant avant la fixation proximale du transmetteur d'image (2) par le support (11) présentant l'oculaire (10), le flexible de sonde (3) étant fixé côté distal avant cette section par rapport au support d'oculaire proximal (11) du transmetteur d'image (2), **caractérisé en ce que** la boucle (15) ou la partie de la boucle (15) s'appuie à l'intérieur du boîtier (16) par endroits sur la paroi intérieure de boîtier, en particulier sur des côtés approximativement opposés, et présente de ce fait une forme différente de la forme circulaire, en particulier une forme ovale.

2. Endoscope selon la revendication 1, **caractérisé en ce que** le support (11) pour le transmetteur d'image (2) se raccorde pratiquement directement à l'extrémité proximale (9) de la section de compensation (14).

3. Endoscope selon la revendication 1 ou 2, **caractérisé en ce que** la fixation (19) du flexible de sonde (3) prévue du côté distal avant la section de compensation ainsi que le support proximal (11) du transmetteur d'image (2) au niveau de l'oculaire (10) font partie du boîtier (16).

4. Endoscope selon une des revendications 1 à 3, **caractérisé en ce que** la boucle (15) ou la boucle partielle (15a) s'étend approximativement dans un plan parallèle à l'extension longitudinale du transmetteur d'image (2).

5. Endoscope selon une des revendications 1 à 4, **caractérisé en ce que** la boucle (15) ou la boucle partielle (15a) du transmetteur d'image (2) est enveloppée par endroits par le flexible de sonde (3).

6. Endoscope selon une des revendications 1 à 5, **caractérisé en ce que** le flexible de sonde (3) se termine au point de fixation côté distal avant la boucle (15) ou la boucle partielle (15a) du transmetteur d'image (2).

7. Endoscope selon une des revendications 1 à 6, **caractérisé en ce que** le boîtier (16) pour le transmetteur d'image (2) présente approximativement sur des côtés opposés une entrée (17) avec fixation du flexible de sonde (3) et une sortie (18) avec support du transmetteur d'image (2) et que les points d'appui sur la paroi intérieure du boîtier (16) se situent approximativement au milieu entre entrée et sortie.

8. Endoscope selon une des revendications 1 à 7, **caractérisé en ce que** le boîtier (16) prévu dans la région de la tête de sonde est formé par un manchon réalisé au moins du côté intérieur dans un matériau permettant le glissement, de préférence en PTFE.

9. Endoscope selon une des revendications 1 à 8, **caractérisé en ce qu'**au moins une courbure du transmetteur d'image (2) dans la section présentant un tracé différent de la forme allongée est fixée thermiquement en position courbe.

10. Endoscope selon une des revendications 1 à 9, **caractérisé en ce que** le transmetteur d'image (2) est composé d'un faisceau image en quartz formé de fibres à gradient d'indice qui présentent chacune un verre de coeur et un verre de gaine et que sur le tracé du transmetteur d'image (2), en particulier dans la section présentant un tracé différent de la forme allongée, est prévue au moins un point d'inflexion en arc (20) dont le rayon de courbure est dimensionné de manière que des rayons lumineux guidés à l'intérieur de la gaine sortent des fibres individuelles vers l'extérieur dans la région du point d'inflexion (20).

11. Endoscope selon la revendication 10, **caractérisé en ce que** le rayon de courbure du faisceau image en quartz se comporte dans la région d'un point d'inflexion en arc (20) par rapport au diamètre des fibres individuelles approximativement comme 100 par rapport à 1 et jusqu'à 5000 par rapport à 1.

12. Endoscope selon la revendication 10 ou 11, **caractérisé en ce que** le point d'inflexion en arc (20) est fixé mécaniquement par un support et/ou thermiquement.

13. Endoscope selon une des revendications 1 à 12, **caractérisé en ce que** sur le tracé du transmetteur d'image (2), de préférence en plus dudit au moins un point d'inflexion en arc (20), est prévu au moins un point de torsion au niveau duquel les fibres individuelles s'étendent en hélice.

14. Endoscope selon la revendication 13, **caractérisé en ce que** le ou les point(s) de torsion se trouve ou se trouvent au niveau d'un point d'inflexion (20), en particulier dans son voisinage immédiat.
